# EUROPÄISCHE PATENTSCHRIFT

(11) **EP 2 550 922 B1**
(45) Veröffentlichungstag und Bekanntmachung des Hinweises auf die Patenterteilung: **14.05.2014**
(21) Anmeldenummer: 12173163.2
(22) Anmeldetag: 22.06.2012
(51) Int. Cl.: A61B 17/80

(54) **Tibiaimplantat zur Straffung der Kniescheibenbänder**
Tibia implant for tightening up patella ligaments
Implant tibial pour fixation des ligaments du genou

(30) Priorität: 26.07.2011 DE 102011079821
(43) Veröffentlichungstag der Anmeldung: 30.01.2013
(73) Patentinhaber: Rita Leibinger GmbH & Co. KG, 78579 Neuhausen o.E. (DE)
(72) Erfinder: Leibinger, Rita, 78570 Mühlheim (DE)
(74) Vertreter: DREISS Patentanwälte PartG mbB

(56) Entgegenhaltungen:
- WO-A1-2012/106323
- DE-A1- 3 224 265
- DE-A1-102005 037 141
- FR-A1- 2 887 760
- US-A- 6 086 593
- US-A1- 2010 076 564

## Beschreibung

Die Erfindung betrifft ein Tibiaimplantat zur Straffung der Kniescheibenbänder.

Aus der US 2010/0076564 A1, welche den nächstkommenden Stand der Technik darstellt, ist ein Implantat bekannt geworden, mit welchem insbesondere in der Veterinärmedizin die Bänder an einem Knie wieder gestrafft werden können. Hierfür wird die Tibia im knienahen Bereich an der Vorderseite von oben bereichsweise aufgespaltet, so dass ein keilförmiger Einschnitt entsteht. Der Tibiaabschnitt darf aber nicht vollständig abgespalten werden. In den einschnitt wird das Implantat eingeschoben, so dass der Tibiaabschnitt nach vorne abgespreizt wird. Das Implantat ist ein Drahtgestellt, welches nach Art einer Leiterbahnstruktur aufgebaut ist und bezüglich des Keilwinkels flexibel ist. Nach dem Einschieben des Implantats in den Tibiaeinschnitt wird dieses so weit aufgebogen, dass sich die Seitenflächen des Implantats an den Schnittflächen des Einschnitts anlegen. Durch Umbiegen von Befestigungslaschen aus der Ebene der Schnittflächen in Richtung auf die Außenoberfläche der Tibia und des Tibiaabschnitts besteht nun die Möglichkeit, das Implantat an der Tibia mittels Schrauben zu befestigen.

Als nachteilig hat sich aber herausgestellt, dass das Implantat viel zu filigran ist und keine Kräfte aufnehmen kann und sich sehr schnell verformt. Außerdem verbindet oder verankert sich das Implantat nur mangelhaft mit dem Knochen.

Die DE 10 2005 037 141 A1 zeigt ein Implantat, welches einen offenporigen Metallschwamm als duroplastischen Knochenersatz aufweist. Die DE 32 24 265 A1 erwähnt ein Verfahren zur Herstellung eines Implantats, bei dem ein Modell aus durchgehend offenzelligem Material verwendet wird, wobei ein Modellmaterial mit einer räumlichen Gitter- oder Netzstruktur zur Anwendung kommt. FR 2 887 760 A1 beschreibt einen Osteotomiekeil, der beispielsweise in eine Tibia oder einen Femur eingesetzt werden kann.

Der Erfindung liegt daher die Aufgabe zugrunde, ein Tibiaimplantat bereit zu stellen, welches formstabiler ist und welches besser mit dem Knochen verwächst.

Diese Aufgabe wird mit einem Tibiaimplantat gelöst, das die Merkmale des Anspruchs 1 aufweist.

Das erfindungsgemäße Implantat weist den wesentlichen Vorteil auf, dass es aufgrund des Schwammgebildes, welches eine beliebige Porengröße aufweisen kann, und insbesondere offenporig ist, sehr steif und formstabil ist und daher sehr hohe Kräfte aufnehmen und abstützen kann. Das Schwammgebilde besitzt bevorzugt eine netzartige, insbesondere regelmäßige Struktur.

Dabei ist die Basisplatte vorteilhaft ein Teil eines umlaufenden, das Schwammgebilde umschließenden Stützrahmens. Einerseits trägt der umlaufende Stützrahmen das Schwammgebilde, andererseits dient der Stützrahmen als stabiler Abstandshalter für den Tibiaabschnitt und hält diesen in der gewünschten abgespreizten Lage, so dass die Bänder ihre neu geschaffene Straffung beibehalten.

Bei einem bevorzugten Ausführungsbeispiel weist das Schwammgebilde eine räumliche Struktur auf. Hierdurch wird gewährleistet, dass die Abstützkräfte in das Innere des Implantats eingeleitet werden, ohne dass das Schwammgebilde beschädigt wird.

Dabei erstreckt sich die Gitterstruktur über mehrere parallele Ebenen. Selbst dann, wenn die Gitterstruktur in einer Ebene eine Fehlstelle aufweist, bleibt die Steifigkeit und Festigkeit erhalten, da die Abstützung durch die weiteren Ebenen unterstützt und gewährleistet wird.

Eine bevorzugte Ausgestaltung des erfindungsgemäßen Tibiaimplantats sieht vor, dass zwei parallele Gitterstrukturebenen um eine halbe Gitterbreite in der x- und/oder in der y-Achse verschoben sind. Dabei sind die Gitterstrecken zwischen den Gitterpunkten in Richtung auf das parallel dazu liegende Gitterstrukturebeneabgeknickt, so dass sie sich in ihren Knickstellen berühren. Die abgeknickten Gitterstrecken spannen nicht nur die Gitterstrukturebene auf sondern versteifen dieses auch gegenüber der parallelen Ebene. Eine detaillierte Beschreibung findet sich weiter unten, wo auf die Zeichnung Bezug genommen wird.

Um das Tibiaimplantat schnell und einfach am Knochen befestigen zu können, ist wenigstens eine vertikale Seitenfläche des Stützrahmens mit wenigstens zwei abragenden Befestigungslaschen versehen. Diese Befestigungslaschen sind flächig ausgebildet, so dass sie in der Laschenebene hohe Kräfte abstützen können. Dabei erstrecken sich die Befestigungslaschen zur Tibia und zum teilabgetrennten Tibiaabschnitt. Eine optimale Anpassung wird dadurch erreicht, dass die Befestigungslaschen horizontal und/oder zur Horizontalen geneigt vom Stützrahmen abragen. Außerdem können die Befestigungslaschen eine oder mehrere Aufnahmeöffnungen für Befestigungsmittel, wie Schrauben oder dergleichen, aufweisen. Eine Verhakung ist ebenfalls möglich. Eine schnelle individuelle Anpassung des Implantats an die jeweilige Knochenform wird dadurch erreicht, dass die Befestigungslaschen biegbar und an die Tibia anpassbar vom Stützrahmen abragen.

Bevorzugt ist das erfindungsgemäße Tibiaimplantat aus Metall, z.B. Edelstahl oder Titan, oder aus Kunststoff, z.B. PEEK, hergestellt ist. Legierungen der Metallwerkstoffe oder Mischungen der Kunststoffe, auch Materialbeimischungen, wie Glasfaser oder Kohlefaser sind denkbar.

Um die Verankerung des Implantats im Knochen zu fördern, weist das Tibiaimplantat eine das Anwachsen von Knochenmasse unerstützende Beschichtung, z.B. Hydroxylapatit, auf. Außerdem wird das Schwammgebilde von Dornen überragt. Diese dringen in die Knochenoberfläche ein und verhindern nein Verrutschen des Tibiaimplantat am Knochen. Dabei sind die Dorne im Bereich der Keilschneide kleiner sind und/oder ragen weniger weit aus dem Schwammgebilde ab als am gegenüber liegenden breiteren Ende. Sehr hohe Kräfte können in bevorzugter Weise dadurch aufgenommen werden, dass die Dorne sich vom einen Schwammgebilde durch das Implantat und dessen Innenraum hindurch zum anderen Schwammgebilde erstrecken und dieses nach außen überragen, so dass die Kräfte vom Tibiaabschnitt direkt auf die Tibia übertragen werden.

Ein schneller Einwachsen des Tibiaimplantats wird vorteilhaft dadurch gefördert, dass der Stützrahmen Durchbrüche aufweist. Durch diese Durchbrüche kann Knochenmasse in das Tibiaimplantat eingeführt werden, so dass der zwischen den Schwammgebilden sich befindende Innenraum schnell zuwächst.

Ein vorteilhafte Herstellungsverfahren sieht vor, dass das Tibiaimplantat mittels eines rapid manufacturing Verfahrens hergestellt wird. Das Implantat kann dadurch ohne große Konstruktionsänderungen in mehreren Größen hergestellt werden.

Weitere Vorteile, Merkmale und Einzelheiten der Erfindung ergeben sich aus den Unteransprüchen sowie der nachfolgenden Beschreibung, in der unter Bezugnahme auf die Zeichnung ein besonders bevorzugtes Ausführungsbeispiel im Einzelnen beschrieben ist. Dabei können die in der Zeichnung dargestellten sowie in der Beschreibung und/oder in den Ansprüchen erwähnten Merkmale jeweils einzeln für sich oder in beliebiger Kombination erfindungswesentlich sein.

In der Zeichnung zeigen:
- Figur 1: eine Frontansicht auf die Vorderseite eine Knies mit in der Tibia sich befindendem Implantat;
- Figur 2: eine Seitenansicht des Knies in Richtung des Pfeils II gemäß Figur 1;
- Figur 3: eine erste perspektivische Ansicht des Tibiaimplantats;
- Figur 4: eine schematische Darstellung des Schwammgebildes;
- Figur 5: eine zweite perspektivische Ansicht des Tibiaimplantats;
- Figur 6: eine Ansicht in Richtung des Pfeils VI gemäß Figur 1 auf die Schneide des Tibiaimplantats;
- Figur 7a: ein Gitterelement in perspektivischer Ansicht; und
- Figur 7b: ein aus mehreren Gitterelementen aufgebautes Netz.

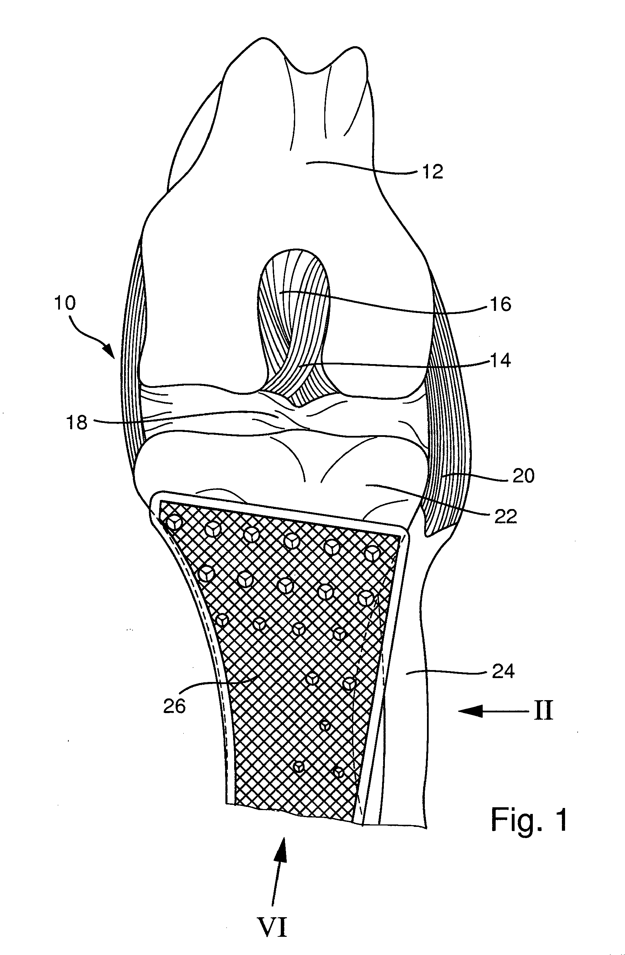

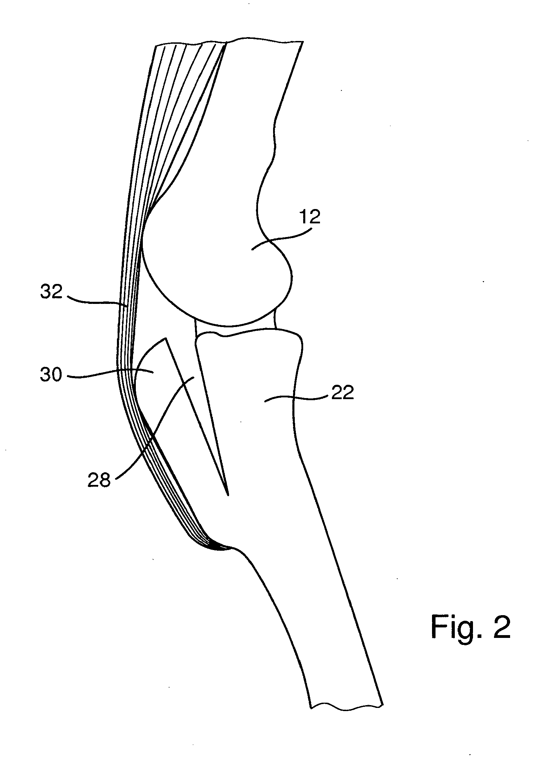

Die Figur 1 zeigt eine Frontansicht auf die Vorderseite eine Knies 10, wobei das untere Ende des Femurs 12. Es sind andeutungsweise das vordere Kreuzband 14 und das hintere Kreuzband 16 sowie die Menisken 18 und das Seitenband 20 dargestellt. Neben der Tibia 22 ist die Fibula 24 zu erkennen. Im knienahen Bereich der Tibia 22 ist ein Tibiaimplantat 26 erkennbar, das einen im Wesentlichen trapezförmigen Querschnitt mit einer konkav geformten Seite aufweist. Dieses Tibiaimplantat 26 befindet sich in einem Einschnitt 28 der Tibia 22, der, wie aus Figur 2 erkennbar, im Wesentlichen vertikal von oben in die Tibia 22 derart eingebracht wird, dass ein Tibiaabschnitt 30 entsteht, dessen unteres Ende noch mit der Tibia 22 verbunden ist. In diesen V-förmigen Einschnitt 28 wird das Tibiaimplantat 26 von der Seite eingesetzt. Durch das Abspreizen des Tibiaabschnitts 30 wird das vordere Band 32 gestrafft.

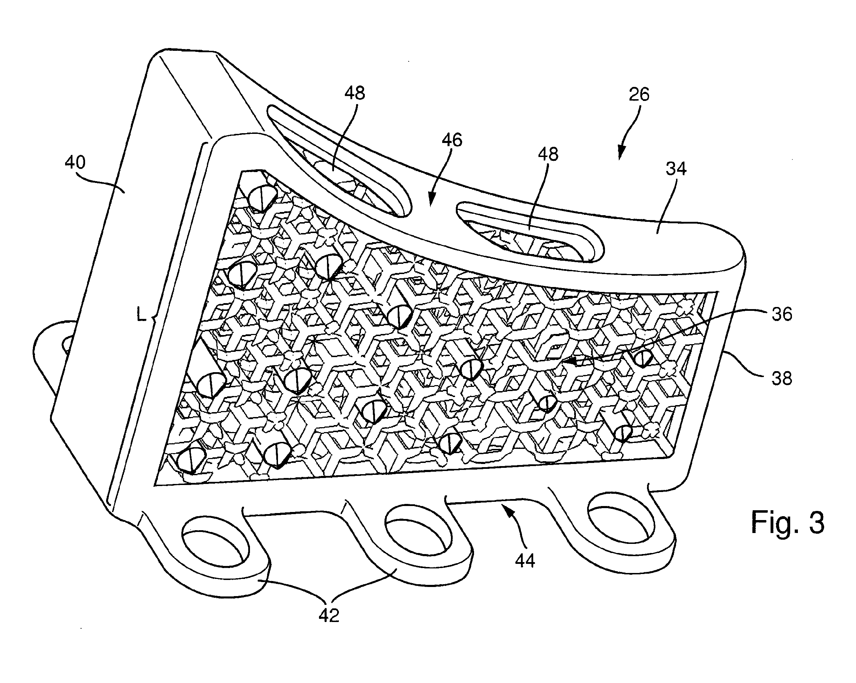

Die Figur 3 zeigt eine perspektivische Ansicht des Tibiaimplantats 26, das einen umlaufenden Stützrahmen 34 und ein dazwischen sich befindendes Schwammgebilde 36 aufweist. Das Tibiaimplantat 26 ist keilförmig ausgebildet und weist eine Keilschneide 38 auf. Die gegenüber liegende Seite 40 besitzt nicht nur eine größere Breite B sondern auch eine größere Länge L. Das Tibiaimplantat 26 weist demnach eine Trapezform auf, wobei die mehrere Befestigungslaschen 42 aufweisende Basisplatte 44 eben und die gegenüber liegende Seite 46 konkav ausgebildet sind. Außerdem ist erkennbar, dass diese konkave Seite 46 mit zwei Durchbrüchen 48 versehen ist, durch welche Knochenmaterial ins Innere des Tibiaimplantats 26 einfüllbar ist. Die Basisplatte 44 kann bei einem alternativen Ausführungsbeispiel auch konkav ausgebildet sein.

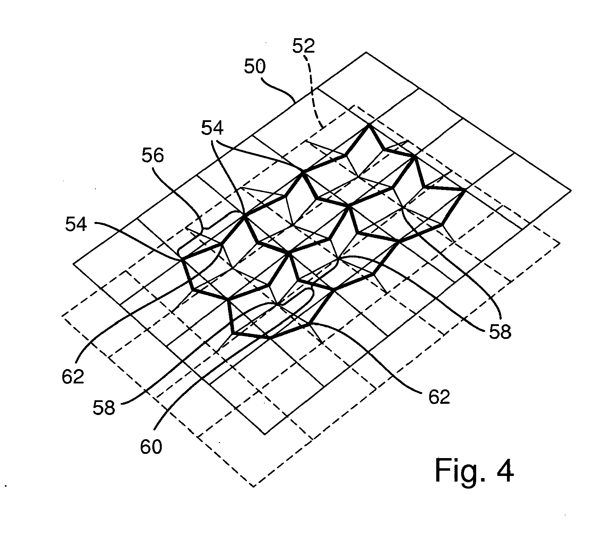

Das Schwammgebilde 36 erstreckt sich über wenigstens zwei Ebenen 50 und 52, was in Figur 4 deutlich zum Ausdruck kommt. Die Ebene 50 ist in der Figur 4 oben und die Ebene 52 darunter angeordnet. Außerdem ist das Gitter in der Ebene 50 um eine halbe Gitterbreite in der x-Richtung und in der y-Richtung verschoben. Die zwischen den Gitterpunkten 54 verlaufenden Gitterstrecken 56 sind in Richtung auf das parallel dazu liegende Schwammgebilde, d.h. die Ebene 52 abgeknickt. Entsprechend sind die zwischen den Gitterpunkten 58 verlaufenden Gitterstrecken 60 in Richtung auf das parallel dazu liegende Schwammgebilde, d.h. die Ebene 50 abgeknickt. Dabei berühren sich die Gitterstrecken 56 und 60 in den Knickstellen 62. Das Schwammgebilde 36 wird somit von einem räumlichen Geflecht gebildet, da eine Vielzahl von Öffnungen aufweist, in welche der Knochen einwachsen kann.

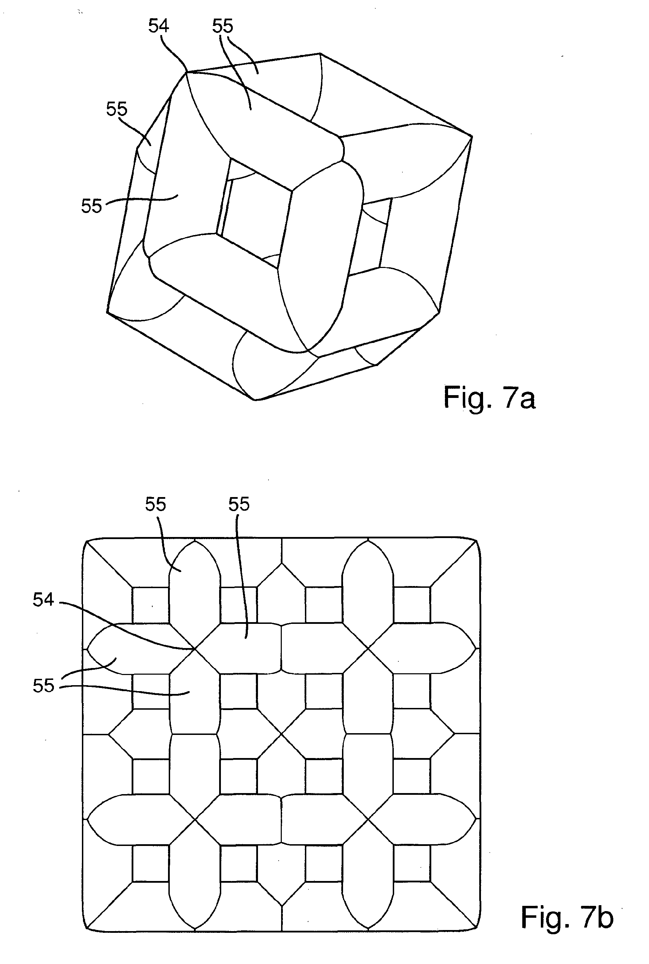

Die Figur 7a zeigt ein einzelnes Gitterelement, welches zwischen 3 Ebenen aufgebaut ist. Vom Gitterpunkt 54 ragen in gleichmäßigen Winkelabständen vier Gitterstäbe 55 zur nächsten, parallelen Ebene ab. Das Gitterelement wird aus zwölf gleichen Gitterstäben 55 aufgespannt. In allen Gitterstäben 55 ist die neutrale Faser gleich. Die Figur 7b zeigt ein aus mehreren Gitterelementen aufgebautes Netz, das sich in der x-y-Ebene im 90°-Winkel erstreckt. In der z-Ebene ist es um 45° verschoben.

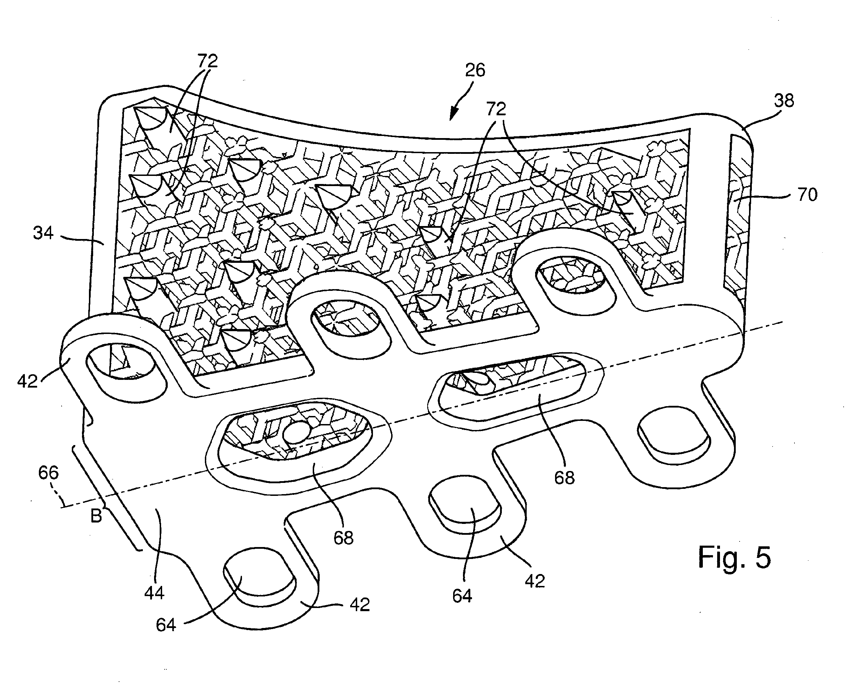

Die Figur 5 zeigt ebenfalls eine perspektivische Ansicht auf das Tibiaimplantat 26, wobei deutlich die Befestigungslaschen 42 erkennbar sind. Sie ragen in der Ebene der Basisplatte 44 vom Stützrahmen 34 ab und weisen jeweils eine Aufnahmeöffnung 64 für eine nicht dargestellte Befestigungsschraube (Knochenschraube) auf, die als quer zur Längsachse 66 des Tibiaimplantats 26 liegendes Langloch ausgebildet ist. Die Basisplatte 44 ist ebenfalls mit Durchbrüchen 68 versehen, so dass auch durch die Basisplatte 44 Knochenmaterial ins Innere des Tibiaimplantats 26 einfüllbar ist.

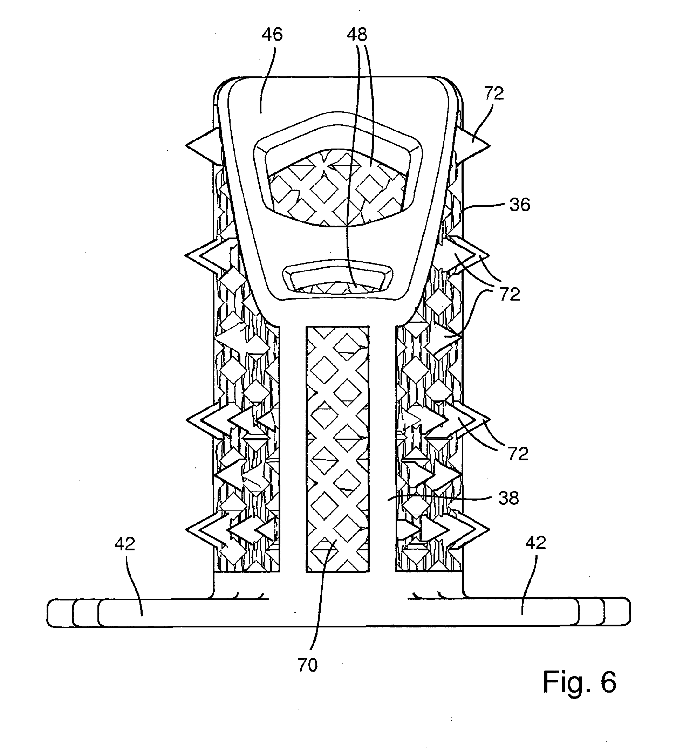

Die Figur 6 zeigt das Tibiaimplantat 26 in Richtung des Pfeils VI gemäß Figur 1, so dass deutlich der Durchbruch 70 in der Keilschneide 38 zu sehen ist. Dieser Durchbruch 70 dient ebenfalls zum Einfüllen von Knochenmaterial ins Innere des Tibiaimplantats 26. Es sind jedoch auch aus dem Schwammgebilde 36 herausragende Dorne 72 erkennbar, die die Außenkontur des Tibiaimplantats 26 überragen. Diese Dorne 72 durchsetzen das gesamte Tibiaimplantat 26, so dass die vom Tibiaabschnitt 30 eingeleiteten Kräfte direkt auf die Tibia 22 übertragen werden. Das Tibiaimplantat 26 und insbesondere das Schwammgebilde 36 wird nicht oder nur minimal belastet. Die Dorne 72 sind im Bereich der Keilschneide 38 nicht nur kürzer sondern weisen auch einen kleineren Durchmesser auf, wodurch das Einschieben des Tibiaimplantats 26 in den Einschnitt 28 erleichtert wird.

## Patentansprüche

1. Tibiaimplantat (26) zur Straffung der Kniescheibenbänder (32), mit einem in Gebrauchslage nach unten sich verjüngenden vertikalen Querschnitt, **dadurch gekennzeichnet, dass** es ein Schwammgebilde (36) mit zwei Seiten aufweist, die an zwei Schnittflächen eines knienahen, im Wesentlichen vertikal von oben in die Tibia (22) eingebrachten Einschnitts (28) anlegbar sind, dass es eine in Längsrichtung der Tibia (22) anordenbare Basisplatte (44) aufweist, welche das Schwammgebilde (36) trägt, und dass das Schwammgebilde (36) zumindest abschnittsweise eine Gitterstruktur aufweist, und dass das Tibiaimplantat (26) einen umlaufenden Stützrahmen (34) aufweist und sich das Schwammgebilde (36) dazwischen befindet.

2. Tibiaimplantat nach Anspruch 1, **dadurch gekennzeichnet, dass** die Basisplatte (44) Teil des umlaufenden, das Schwammgebilde (36) umgebenden Stützrahmens (34) ist.

3. Tibiaimplantat nach einem der vorhergehenden Ansprüche, **dadurch gekennzeichnet, dass** das Schwammgebilde (36) offenporig ist und/oder eine räumliche Struktur aufweist und/oder von Dornen (72) überragt wird.

4. Tibiaimplantat nach einem der vorhergehenden Ansprüche, **dadurch gekennzeichnet, dass** die Gitterstruktur sich in einer Ebene erstreckt und das Schwammgebilde (36) mehrere parallele Ebenen (50, 52) derartiger Gitterstrukturen aufweist.

5. Tibiaimplantat nach einem der vorhergehenden Ansprüche, **dadurch gekennzeichnet, dass** zwei parallele Gitterstrukturebenen um eine halbe Gitterbreite in der x- und/oder in der y-Achse verschoben sind.

6. Tibiaimplantat nach Anspruch 5, **dadurch gekennzeichnet, dass** die Gitterstruktur von Gitterstrecken (56, 60) und Gitterpunkten (54, 58) gebildet wird, wobei die Gitterstrecken (56, 60) zwischen den Gitterpunkten (54, 58) in Richtung auf die parallel dazu liegende Gitterstrukturebene abgeknickt sind, so dass die Ebenen sich in ihren Knickstellen (62) berühren.

7. Tibiaimplantat nach einem der vorhergehenden Ansprüche, **dadurch gekennzeichnet, dass** wenigstens die vertikale Basisplatte (44) des Stützrahmens (34) mit wenigstens zwei abragenden Befestigungslaschen (42) versehen ist.

8. Tibiaimplantat nach Anspruch 7, **dadurch gekennzeichnet, dass** sich die Befestigungslaschen (42) zur Tibia (22) und zum teilabgetrennten Tibiaabschnitt (30) erstrecken.

9. Tibiaimplantat nach Anspruch 7 oder 8, **dadurch gekennzeichnet, dass** die Befestigungslaschen (42) horizontal und/oder zur Horizontalen geneigt vom Stützrahmen (34) abragen.

10. Tibiaimplantat nach einem der Ansprüche 7 bis 9, **dadurch gekennzeichnet, dass** die Befestigungslaschen (42) eine oder mehrere Aufnahmeöffnungen (64) für Befestigungsmittel, wie Schrauben oder dergleichen, aufweisen.

11. Tibiaimplantat nach einem der vorhergehenden Ansprüche, **dadurch gekennzeichnet, dass** es aus Metall, z.B. Edelstahl oder Titan, oder aus Kunststoff, z.B. PEEK, hergestellt ist und/oder eine das Anwachsen von Knochenmasse unerstützende Beschichtung, z.B. Hydroxylapatit, aufweist.

12. Tibiaimplantat nach Anspruch 3, **dadurch gekennzeichnet, dass** die Dorne (72) sich in Richtung des verjüngenden Querschnitts verkleinern und/oder weniger weit aus dem Schwammgebilde (36) abragen.

13. Tibiaimplantat nach Anspruch 3 oder 12, **dadurch gekennzeichnet, dass** die Dorne (72) sich von der einen, an der Tibia (22) anliegenden Seite durch das Tibiaimplantat (26) hindurch zur anderen, an der Tibia (22) anliegenden Seite erstrecken.

14. Tibiaimplantat nach einem der vorhergehenden Ansprüche, **dadurch gekennzeichnet, dass** die Basisplatte (44) oder ein das Schwammgebilde (36) umgebender Stützrahmen (34) Durchbrüche (48, 68, 70) aufweist.

15. Verfahren zur Herstellung eines Tibiaimplantats nach einem der vorhergehenden Ansprüche, **dadurch gekennzeichnet, dass** es mittels eines rapid manufacturing Verfahrens hergestellt wird, indem mehrere zumindest abschnittsweise insbesondere regelmäßige Gitterstrukturebenen mit Gitterstrecken (56, 60) und Gitterpunkten (54, 58) hergestellt werden, wobei sich die Gitterstrecken (56, 60) in Richtung auf die parallel dazu liegende Gitterstrukturebene abgeknickt sind, so dass die Ebene sich in den Knickstellen (62) berühren.

## Claims

1. Tibia implant (26) for tightening up the patella ligaments (32), having a vertical cross-section which tapers in the downward direction in the in-use position, **characterised in that** it has a sponge formation (36) having two sides which can be applied to two cut faces of an incision (28) close to the knee which is made substantially vertically into the tibia (22) from above, **in that** it has a base plate (44) which can be arranged in the longitudinal direction of the tibia (22) and which carries the sponge formation (36), and **in that** at least a portion or portions of the sponge formation (36) have a lattice structure, and **in that** the tibia implant (26) has a supporting frame (34) extending round in a loop and the sponge formation (36) is situated therewithin.

2. Tibia implant according to claim 1, **characterised in that** the base plate (44) is part of the supporting frame (34) extending round in a loop which surrounds the sponge formation (36).

3. Tibia implant according to either of the preceding claims, **characterised in that** the sponge formation (36) is open-pored and/or has a three-dimensional structure and/or has spigots (72) projecting beyond it.

4. Tibia implant according to one of the preceding claims, **characterised in that** the lattice structure extends in a plane and the sponge formation (36) has a plurality of parallel planes (50, 52) of lattice structure of this kind.

5. Tibia implant according to one of the preceding claims, **characterised in that** two parallel planes of lattice structure are offset on the x-axis and/or the y-axis by half the lattice pitch.

6. Tibia implant according to claim 5, **characterised in that** the lattice structure is formed by lattice lines (56, 60) and lattice points (54, 58), the lattice lines (56, 60) being kinked between the lattice points (54, 58) in the direction of the plane of lattice structure lying parallel thereto, thus causing the planes to touch at their kinked points (62).

7. Tibia implant according to one of the preceding claims, **characterised in that** at least the vertical base plate (44) of the supporting frame (34) is provided with at least two projecting fastening lugs (42).

8. Tibia implant according to claim 7, **characterised in that** the fastening lugs (42) extend to the tibia (22) and to the partly severed portion (30) of the tibia.

9. Tibia implant according to claim 7 or 8, **characterised in that** the fastening lugs (42) project from the supporting frame (34) horizontally and/or at an inclination to the horizontal.

10. Tibia implant according to one of claims 7 to 9, **characterised in that** the fastening lugs (42) have one or more receiving openings (64) for fastening means such as screws or the like.

11. Tibia implant according to one of the preceding claims, **characterised in that** it is made of metal, e.g. stainless steel or titanium, or of plastics material, e.g. PEEK, and/or has a coating, e.g. hydroxylapatite, which assists the ingrowth of bone mass.

12. Tibia implant according to claim 3, **characterised in that**, in the direction in which the cross-section tapers, the spigots (72) become smaller and/or project less far from the sponge formation (36).

13. Tibia implant according to claim 3 or 12, **characterised in that** the spigots (72) extend from one side which rests against the tibia (22) through the tibia implant (26) to the other side which rests against the tibia (22).

14. Tibia implant according to one of the preceding claims, **characterised in that** the base plate (44) or a supporting frame (34) surrounding the sponge formation (36) has perforations (48, 68, 70).

15. Method of producing a tibia implant according to one of the preceding claims, **characterised in that** it is produced by a rapid manufacturing method by producing a plurality of planes of lattice structure of which at least a portion or portions are, in particular, regular and which have lattice lines (56, 60) and lattice points (54, 58), the lattice lines (56, 60) being kinked in the direction of the plane of lattice structure lying parallel thereto, thus causing the planes to touch at the kinked points (62).

## Revendications

1. Implant tibial (26) pour renforcer les ligaments du genou (32), ayant une coupe transversale verticale effilée vers le bas en position d'utilisation, **caractérisé en ce qu'**il présente une entité en éponge (36) ayant deux côtés, qui peuvent être agencés sur deux surfaces générées par découpe d'une incision (28) située à proximité du genou, et réalisée sensiblement verticale depuis le dessus dans le tibia (22), **en ce qu'**il présente une plaque de base (44) agencée dans la direction longitudinale du tibia (22), qui supporte l'entité en éponge (36), et **en ce que** l'entité en éponge (36) présente au moins partiellement une structure en treillis, et **en ce que** l'implant tibial (26) présente un cadre de support périphérique (34) dans lequel se trouve l'entité en mousse (36).

2. Implant tibial selon la revendication 1, **caractérisé en ce que** la plaque de base (44) et une partie du cadre de support périphérique (34) entourant l'entité en éponge (36).

3. Implant tibial selon l'une quelconque des revendications précédentes, **caractérisé en ce que** l'entité en éponge (36) est à cellules ouvertes, et/ou présente une structure spatiale, et/ou va faire saillie via des pointes (72).

4. Implant tibial selon l'une quelconque des revendications précédentes, **caractérisé en ce que** la structure en treillis s'étend dans un plan, et l'entité en éponge (36) présente plusieurs structures en treillis dans de tels plans parallèles (50, 52).

5. Implant tibial selon l'une quelconque des revendications précédentes, **caractérisé en ce que** deux plans de structure en treillis parallèles sont décalés d'une demie largeur de treillis, dans la direction de l'axe x et/ou de l'axe y.

6. Implant tibial selon la revendication 5, **caractérisé en ce que** la structure en treillis va être constituée de lignes de treillis (56, 60) et de points de treillis (54, 58), de telle sorte que les lignes de treillis (56, 60), entre les points de treillis (54, 58), sont pliées en direction du plan de structure de treillis s'étendant parallèlement à celles-ci, de telle sorte que les plans se rejoignent au niveau de leurs pliures (62).

7. Implant tibial selon l'une quelconque des revendications précédentes, caractérisé en qu'au moins la plaque de base verticale (44) du cadre de support (34) est prévue ayant au moins deux languettes de fixation (42) faisant saillie.

8. Implant tibial selon la revendication 7, **caractérisé en ce que** les languettes de fixation (42) s'étendent vers le tibia (22) et vers un tronçon de tibia (30) partiellement séparé.

9. Implant tibial selon la revendication 7 ou 8, **caractérisé en ce que** les languettes de fixation (42) s'étendent horizontalement, et/ou inclinées par rapport à l'horizontale depuis le cadre de support (34).

10. Implant tibial selon l'une quelconque des revendications 7 à 9, **caractérisé en ce que** les languettes de fixation (42) présentent une ou plusieurs ouvertures de réception (64) destinées à des moyens de fixation, comme des vis ou analogues.

11. Implant tibial selon l'une quelconque des revendications précédentes, **caractérisé en ce qu'**il est réalisé en métal, par exemple de l'acier inoxydable ou du titane, ou en matière plastique, par exemple du PEEK, et/ou comporte un revêtement de soutien de la croissance de la masse osseuse, comme de l'hydroxyapatite.

12. Implant tibial selon la revendication 3, **caractérisée en ce que** les pointes (72) rétrécissent dans la direction de la coupe transversale effilée et/ou font moins saillie de l'entité en éponge (36).

13. Implant tibial selon la revendication 3 ou 12, **caractérisée en ce que** les pointes (72) s'étendent depuis un premier côté adjacent au tibia (22), au travers de l'implant tibial (26), vers un autre côté adjacent au tibia (22).

14. Implant tibial selon l'une quelconque des revendications précédentes, **caractérisé en ce que** la plaque de base (44) ou un cadre de support (34) entourant l'entité en éponge (36) présente des ouvertures traversantes (48, 68, 70).

15. Procédé pour fabriquer un implant tibial selon l'une quelconque des revendications précédentes, **caractérisé en ce qu'**il va être fabriqué au moyen d'un procédé de fabrication rapide, dans lequel plusieurs plans de structure en treillis en particulier réguliers, au moins partiellement, vont être mis en oeuvre à l'aide de lignes de treillis (56, 60) et de points de treillis (54, 58), dans lequel les lignes de treillis (56, 60) sont pliées en direction du plan de structure en treillis s'étendant parallèlement à celles-ci, de telle sorte que les plans se rejoignent au niveau de leurs pliures (62).
